**Europäisches Patentamt**

**European Patent Office**

**Office européen des brevets**

(19)

(11) Veröffentlichungsnummer: **0 068 513**
**A1**

(12) # EUROPÄISCHE PATENTANMELDUNG

(21) Anmeldenummer: **82105888.0**

(22) Anmeldetag: **01.07.82**

(51) Int. Cl.³: **A 61 K 35/26**
**// (A61K35/26, 35/23)**

(30) Priorität: **01.07.81 EP 81105110**

(43) Veröffentlichungstag der Anmeldung: **05.01.83**
**Patentblatt 83/1**

(84) Benannte Vertragsstaaten: **AT CH FR GB IT LI SE**

(71) Anmelder: **Selzer, Hans, Dr., Schönmünzach, D-7292 Baiersbronn 9 (DE)**

(72) Erfinder: **Selzer, Hans, Dr., Schönmünzach, D-7292 Baiersbronn 9 (DE)**

(74) Vertreter: **Freiherr von Welser, Hubert, Danziger Strasse 15, D-8000 München 40 (DE)**

(54) **Heilmittel aus Lymphe von Säugetieren.**

(57) Heilmittel für Humantherapie, das aus der Lymphe aus dem Ductus thoracicus von Säugetieren unter Ausfiltern aller grobmolekularen und zellulären Bestandteile mit einem Filter von 8 μm mittlerer Porenweite gewonnen wird und das nurmehr reine Lymphflüssigkeit und die kleinen im Ductuc thoracicus rezirkulierenden Lymphozyten enthält und das zur intravenösen. intramuskulären oder subcutanen Injektion bei Patienten zur Erzielung adoptiver Immunität. insbesondere gegen Krebs- oder Autoimmunerkrankungen bestimmt ist. Zur Verstärkung der Wirkung des Heilmittels können ihm ausgefilterte Extrakte der Nebenniere des gleichen Spendertieres zugesetzt sein.

ACTORUM AG

HUBERT FREIHERR VON WELSER

RECHTSANWALT

ZUGELASSEN AN DEN LANDGERICHTEN MÜNCHEN I UND II
AM OBERLANDESGERICHT MÜNCHEN UND AM
BAYERISCHEN OBERSTEN LANDESGERICHT

DANZIGER STRASSE 15
8000 MÜNCHEN 40
TELEFON (089) 36 70 22

0068513

## Beschreibung der Erfindung

Die Erfindung betrifft ein Heilmittel aus Lymphe
von Säugetieren für die Humantherapie sowie ein
Verfahren zu seiner Herstellung.

Bekannt ist, aus lymphatischen Organen von Säugetieren
Extrakte zu gewinnen, z. B. aus dem Thymus oder
der Milz (z. B. DE-AS 2 110 436), es werden
dabei aber nicht die lebenden Lymphzellen benutzt,
ebenso wenig wie bei der Serumtherapie, die sich
allein auf die Verwendung der von dem Lymphsystem
des Spendertieres erzeugten Antikörper beschränkt.
Die Abtrennung der Lymphzellen und anderer
zellulärer und großmolekularer Bestandteile
begründete sich in der Gefahr von Immunreaktionen
oder Überempfindlichkeitsreaktionen des Abwehrsystems
des Empfängers gegen die ihm übertragenen Lymphbestandteile

BANKKONTO: DEUTSCHE BANK AG MÜNCHEN 22/26 843 (BLZ 700 700 10) · POSTSCHECKKONTO MÜNCHEN 1999 94-803
ANDERKONTO: DEUTSCHE BANK AG MÜNCHEN 22/26 850 (BLZ 700 700 10)

BAD ORIGINAL

0068513

- 4 -

In US-PS 3 719 182 und DE-OS 2 416 842 werden Verfahren
beschrieben, mittels einer an Patienten im Milchbrustgang angelegten Fistel über längere Zeitabschnitte
nach Möglichkeit die gesamte Lymphproduktion
zu entnehmen, daraus die Antikörper und Lymphzellen
durch Zentrifugieren abzuschneiden und die Lymphflüssigkeit und die Lymphzellen unter Umständen
nach Reinigung dem Körper des Patienten zurückzugeben.
Durch die dauernde Entnahme von Lymphozyten wird
deren ständige Neuproduktion erzwungen. Durch
Ansammlung der entnommenen Lymphozyten und deren
Rückgabe in den Kreislauf des Patienten soll dessen
Immunantwort verstärkt werden.

Diese Verfahren haben in der ärztlichen Praxis
keine Bedeutung erlangt, da einerseits die Erfolge
zweifelhaft waren, zum anderen der Zugang zum
Ductus thoracicus einen erheblichen operativen
Eingriff erfordert. Ein Erfolg ist schon deshalb
in Frage gestellt, weil ein erfolgreicher Immunangriff
auf einen Tumor nicht allein eine Quantitativsondern vor allem eine Qualitätsfrage ist.

Es kann davon ausgegangen werden, daß dauernd im
Organismus des Menschen Krebszellen entstehen,
allein schon im Hinblick auf die vielen der heutigen
Erkenntnis zugänglichen Ursachen und Entstehungsmöglichkeiten von krebsartigen Erkrankungen.

0068513

- 5 -

BAD ORIGINAL

Aus der Tatsache, daß bei einem Idividuum maligne
Tumore ungestört wachsen können, unter den gleichen
Bedingungen und Voraussetzungen bei anderen aber
nicht, kann geschlossen werden, daß die Entwicklung
von Tumoren Folgen eines Immundeffektes bei dem
befallenen Individuum ist. Es gibt eine Reihe von
Möglichkeiten eines solchen Immundefektes, denen
aber gemeinsam ist, daß das Immunsystem die entstehenden Krebszellen nicht bzw. nicht als solche
erkennen kann und sie daher toleriert. In einem
solchen Falle muß eine systematische Vermehrung
der eigenen Lymphozyten erfolglos bleiben, d. h.
in den wesentlichen Fällen der Toleranz oder
des Nichterkennenkönnens von spezifischen Antigenen
in den Membranen der Krebszellen.

Andererseits kann, wie das bei soliden Tumoren
der Fall ist, bei einer großen Menge von Krebszellen
eine Immunabwehr praktisch unmöglich werden, weil
eine Bereitstellung einer ausreichenden Lymphozytenkonzentration in der Region des Tumors kaum möglich
erscheint.[1] Nach derzeitiger Lehre muß daher zunächst
die Masse des Krebsgewebes drastisch durch chirurgischen
Eingriff, Bestrahlung oder Chemotherapie reduziert
werden, wobei gerade die letzteren beiden Eingriffe
kaum ohne weitergehende Schädigung des ohnehin als
defekt erwiesenen Immunsystemes möglich sind,
vor allem wird durch keine dieser Maßnahmen die

([1]Jungermann und Möller: "Biochemie", Springerverlang 1980

0068513

Ursache des patogenen Geschehens getroffen, nämlich
die Insuffizienz der eigenen Immunabwehr, sodaß die
Bildung von Metastasen und Rezidiven nicht verhindert
werden kann.

Bei Autoimmunerkrankungen ist eine Behandlung zur
Erzeugung einer zellulären adoptiven Immunität
nicht bekannt geworden. Im Gegenteil stehen hier
im wesentlichen nur Verfahren der Suppression
des Immunsystemes des Patienten zur Verfügung,
soweit man von der vollständigen letalen Suppression
dieses Immunsystemes durch Bestrahlung und nachträgliche
Wiederzuführung von gesundem Knochenmark eines
Spenders absieht. Dieses Verfahren kann nur sehr
entfernt und allgemein mit dem erfindungsgemäßen
in Zusammenhang gebracht werden und zudem ist
es auf Mark syngener Spender beschränkt. Es ergibt
sich daher  als Aufgabenstellung, das körpereigene
Immunsystem nicht nur quantitativ, wie dies
mit den oben genannten Druckschriften vorgeschlagen
wird, sondern zugleich auch qualitativ, wobei beides
gleich wichtig wäre, zu ergänzen und zu verstärken,
d. h.bei krebsartigen Erkrankungen die fehlenden
tumorspezifisch sensibilisierten  kompetenten
Lymphozyten adoptiv unmittelbar im Zielbereich,
z. B. in dem den Tumor umgebenden Gewebe in ausreichender
Menge zur Verfügung zu stellen.

- 7 -

Hinzukommt, daß jede Krebserkrankung, d. h. eine
Entwicklung von malignem Tumorgewebe nach Durchbrechen
oder Umgehen der Immunabwehr oder deren Inkompetenz
eine allgemeine Schwächung des Patienten hervorruft
und durch diese Streßsituation wiederum  die Immunabwehr in einem circulus vitiosus beeinträchtigt
wird. Es ist daher eine weitere Aufgabe der Erfindung,
gerade diesem Effekt durch Regeneration des Immunsystems des Patienten entgegenzuwirken.

Schließlich ist es notwendig, eine Selektionierung
der zellulären Bestandteile der Spenderlymphe vorzunehmen,
um einerseits deren spezifisch wirksame Bestandteile
zu erhalten und um eine craft vs. host-Reaktion
des Immunsystemes des Empfängers auszuschließen.
Zu den spezifisch wirksamen Bestandteilen müssen
diejenigen Zellen gerechnet werden, die selbständig
eine Immunantwort, sowie diejenigen, die eine
kompetente Antikörperproduktion in die Wege leiten
können. Ausgeschieden müssen vor allem die
großmolekularen Stoffe und großen Zellen werden,
bei denen die Gefahr einer Immunreaktion des Patienten
bestehen würde. Dabei muß die  Selektion so schonend
wie möglich vorgenommen werden, um eine Schädigung
der zu übertragenden Zellen auszuschließen und deren
volle Funktion und Lebensfähigkeit zu erhalten.

- 8 -

Diese Aufgaben wurden dadurch gelöst, daß
das Heilmittel aus aus der Lymphe des Spendertieres gewonnener von allen einen Filter
von mittlerer Porenweite von 8 μm nicht
passierenden grobmolekularem und zellulärem
Material gereinigten und nur die im Ductus
thoracicus des Spendertieres rezirkulierenden
kleinen Lymphozyten enthaltenden Lymphflüssigkeit
besteht.

Versuchsreihen mit Säugetieren und in der Humantherapie haben gezeigt, daß die wirksame Substanz
die kleinen Lymphozyten sind, die ebenso wie die
gereinigte Lymphflüssigkeit keine erkennbaren
Patienten oder Heilverfahren belastende Immunantworten hervorrufen, was auf  die Reinigung
der Lymphflüssigkeit von grobmolekularen und
anderern Bestandteilen als den kleinen T- und B-
Lymphozyten zurückzuführen ist. Die kleinen
Lymphozyten sind ausgereifte und kompetente
T-Lymphozyten, insbesondere Helfer- und Killerzellen,
die z. B. Tumore unmittelbar angreifen sowie
vermutlich auch Suppressorzellen, von denen
anzunehmen ist, daß sie bei der Bekämpfung
von Autoimmunkrankheiten entscheidend mitwirken.
Zu den kleinen Lymphozyten zählen auch die
in geringerem Umfang aus dem Ductus Thoracicus
gewinnbaren  kleinen, ausgereiften und kompetenten
B-Lymphozyten, die eine adoptive Antikörperproduktion
bewirken können.

- 9 -

Es hat sich als vorteilhaft erwiesen, dem Heilmittel
die gereinigten Extrakte der Nebenniere des gleichen
Spendertieres zuzusetzen, da einerseits bei den
für die Therapie mit dem erfindungsgemäßen spezifischen
Erkrankungen dem Patienten Nebennierenwirkstoffe
in erheblichem Umfang fehlen, andererseits diese
Wirkstoffe eine, wie Versuche erwiesen haben,
aktivierende Wirkung auf das adoptive und eigene
Immunsystem des Patienten haben.

Das Herstellungsverfahren besteht erfindungsgemäß
darin, daß die Lymphe aus dem Ductus thoracicus
des Spendertieres entnommen und durch einen Filter
mit mittlerer Porenweite von 8 mm bei einem
Druck von 0,3 bis 0,6 bar gefiltert wird.
Diese Filterung muß möglichst schonend erfolgen,
um die gewonnenen Lymphozyten nicht zu  schädigen,
was bei bekannten Verfahren zur Trennung der
einzelnen Lymphozytenklassen zu befürchten war
(z. B. langfristiges Schütteln oder Zentrifugieren).
Die angegebene Porenweite gewährleistet, daß alle
anderen Lymphozytenklassen ausgeschieden werden,
die durchweg größer sind und gegen deren Übertragung wie z.B bei Granulozyten und Mikrofagen
begründete Bedenken bestehen. Ferner müssen alle in
der Lymphe enthaltenen grobmolekularen Stoffe
sowie Zellen und Zellbruchstücke zurückgehalten werden.

0068513

BAD ORIGINAL

Die erhaltenen Lymphozyten sind durchwegig langlebige
Zellen, sodaß eine längere Standzeit des Heilmittels
möglich ist.

Die zuzusetzenden Nebennierenextrakte werden ohne
Anwendung von Hitze oder chemischen Verfahren aus
den Nebennieren des gleichen Spendertieres gewonnen
und durch ein alle grobmolekularen und zellulären
Substanzen zurückhalten des Filters gefiltert und
die so gereinigten Extrakte dem Heilmittel zugesetzt.

Es wurde gefunden, daß das erfindungsgemäße Heilmittel
erfolgreich gegen Krebserkrankungen, auch in deren
fortgeschrittenen Stadien, insbesondere auch zur
Bekämpfung von Metastasen und Rezidiven eingesetzt
werden kann und daß es bei Autoimmunkrankheiten
sonst nicht erreichbare Erfolge, ebenfalls auch
in fortgeschrittenen Stadien erbringen kann.

Diese Heilerfolge waren vor allem deswegen überraschend,
weil gegen die Verwendung von Allolmyphozyten und
vor allem solcher tierischer Herkunft Bedenken
und Vorurteile bestanden, deren Unbegründetheit sich
bei dem erfindungsgemäßen Heilmittel aber erwiesen
hat.

0068513

Die Vermittlung von adoptiver Immunität durch
Übertragung von Fremdlymphozyten von Tier zu Tier
ist in der Praxis der biochemischen und mikrobiologischen Forschung an sich durchaus geläufig.
Jedoch beschränkt man sich auch hier, wenn man
die craft vs. host-Reaktion vermeiden will,
auf die Verwendung von Zellen eines syngenen
Spenders[2]. In der Humanmedizin bestanden und
bestehen naheliegende und ernst zu nehmende Bedenken
aus dem gleichen Grund, sodaß grundsätzlich nur die
eingangs zitierte Rückübertragung von Eigenlymphozyten
in der Praxis gebräuchlich war.

Es bestanden des weiteren Bedenken gegen die Kompetenz
von Tierlymphozyten für Antigene humaner Tumoren
oder für autoimmune Erkrankungen.

Die an sich bekannte Entnahme von Lymphflüssigkeit
aus dem Ductus thoracicus bewirkt bereits eine
Selektion der Lymphozyten. Diese sind dort im
wesentlichen kleine T- und B-Lymphozyten, da nur
diese in den Blut- und Lymphbahnen rezirkulieren,
wobei die T-Lymphozyten den weit überwiegenden
Anteil ausmachen.[3] Kleine Lymphozyten mit einem
Durchmesser von etwa 8μm sind bereits ausgereift
und sensibilisiert. Große nicht sensibilisierte
Lymphozyten treten erst bei längerer Ductus Thoracicus-
Trainage auf, nachdem die kleinen ausgereiften

([2]Bier, Götze u. a.: "Experimentelle und klinische
Immunologie", Springer Verlang 1979 Seite 35 rechte Spalte unter
([3]Benacerraf, Unanue: "Immunologie", Verlag de Gruyter 1982
    Seite 145

- 12 -

Lymphozyten weitgehend aus dem Kreislauf entnommen
sind. Eine solche Dauertrainage ist im erfindungsgemäßen
Verfahren nicht vorgesehen, sondern nur eine einmalige
Entnahme aus einem intakten Tier.

Die Fraktionierung der erhaltenen Lymphzellen und
ihr Abtrennen durch die vorgeschlagene Filterung
von den übrigen zellulären und makromolekularen
Bestandteilen der entnommenen Lymphe bewirkt,
daß ausschließlich die kleinen T- und B-Lymphozyten
erhalten werden, da nur deren Durchmesser den
Betrag von 8µm nicht übersteigt. Außerdem bewirkt
die Entnahme aus dem Ductus thoracicus, daß ohnehin
aus der Population der weißen Zellen nur die
zirkulierenden Lymphozyten und von diesen vorwiegend
T-Lympozyten erhalten werden.

Eine Erklärung der in den nachstehenden Beispielen
belegte Wirkungsweise ist insofern schwierig und
muß in entscheidenden Zusammenhängen hypothetisch
bleiben, da die mikrobiologische Funktion dieser
Lymphozyten und ihre Rolle in Zusammenwirken und
in der Steuerung des Lymphsystemes in weiten
Bereichen seinerseits nur in Hypothesen bekannt ist.
Man kann aber bei der Tumortherapie zunächst davon
ausgehen, daß zumindesten bei bestimmten Tumortypen
sowohl bei Säugetieren wie bei Menschen ein Bestand
gleicher Membranantigene neben spezifischen

- 13 -

0068513

dem jeweiligen Träger des Tumors zugehörigen
Zellwandantigenen vorliegen, wie dies auch
im Verhältnis zwischen  Humantumoren gleichen Typs
gegeben ist. Hieraus folgt die Möglichkeit,
daß im gesunden Immunsystem des Spendertieres
für diese tumorspezifischen Antigene kompetente
T-Lymphozyten, gegebenenfalls auch B-Lymphozyten
vorliegen, die die fehlenden kompetenten Zellen
des Patienten subsituieren können. Hierdurch
ergibt sich die Ergänzung des defekten Abwehrsystemes des Patienten und somit die Behebung
des qualitativen Immundefektes.  Der bestehende
quantitative Defekt,  der allgemein dadurch zustande
kommt, daß das Tumorgewebe und dessen Umgebung
für Lymphozyten bei deren geringer Wandergeschwindigkeit im Gewebe außerhalb der Blut- und Lymphbahnen
schwer und daher nur unzureichend zugänglich ist,
kann dadurch behoben werden, daß mit dem erfindungsgemäßen Mittel ein Umspritzen des Tumorgewebes
möglich ist. Dadurch können an dem eigentlichen Ort
des patogenen Geschehens kompetente Lymphozyten
in großer Zahl bereitgestellt werden.

Es kann ferner angenommen werden, daß durch die
von den sich an die Tumorantigene bindenden
Spender-T-Lymphozyten abgegebenen Mediatorstoffen[4]

(4 Benacerraf a. a. O. Seite 145 fl. und 148

- 14 -

nicht nur die mit ihnen übertragenen B-Lympozyten
zur Mitose, Umwandlung in Plasmazellen und Proliferation
von Antikörpern angeregt werden, sondern auch, daß
diese den spezifischen Tumorantigenen entsprechenden
Mediatoren auf die B-Lymphozyten des patienteneigenen Immunsystemes anregen und die dort fehlende
humorale Abwehr des Patienten in Gang setzen und
ebenso dessen Makrophagen aktivieren[5].

Es kann nicht ausgeschlossen werden, daß eine
craft vs. host-Reaktion des patienteneigenen Immunsystemes eintritt. Doch ist vermutlich hierbei ein sehr
einfaches      Mengenproblem maßgebend und man kann
davon ausgehen, daß im Bereich des Tumors, in dem
die Spenderlymphozyten injiziert wurden, keine
ausreichende Anzahl von T-Lymphozyten des ohnehin
geschwächten Patientenimmunsystems anwesend sind,
sodaß zunächst mit einer relativen Toleranz gerechnet
werden kann. Bei der sehr langsamen Wanderbewegung
der Lymphozyten im Gewebe wird es längere Zeit
dauern, bis eine solche craft vs. host-Reaktion
in den gewünschten Effekt wirksam störender Weise
eintritt, aber bis dahin haben die Spenderlymphozyten,
soweit sie Helfer- bzw. Killerzellen sind, ihre
Aufgabe erfüllt und es kann durch erneute und damit
massenhafte Dotierung mit Spenderlymphozyten das
unter Umständen erreichte Gleichgewicht zwischen
dem Suppressoreffekt des Immunsystemes des Wirts
mit den Fremdlymphozyten wieder zugunsten der Therapie

([5] vgl. Benacerraf, Unanue a. a. O. Seite 151 fl.
"Allogener Effekt".

- 15 -

0068513

aufgehoben werden, wie andererseits die von den
Spender-B-Lymphozyten herrührenden für die Krebsantigene kompetenten Antikörper kaum angegriffen
werden.

Die Zugabe von Nebennierenextrakten erscheint
zunächst deswegen problematisch, als solche
Extrakte, insbesondere Kortikostereoide im
Gegenteil zur Immunsuppression bei Autoimmunerkrankungen verwendet werden[6]. Diese Therapie
zielt jedoch auf und wirkt gegen zirkulierende
Antikörper und Granulozyten und erfaßt daher
nicht die hier wesentliche lymphozytische
Aktivität. Andererseits ist es eine wesentliche
Funktion der von den Nebennieren abgegebenen
Substanzen, Streßsituationen zu überwinden.
Solche Streßbedingungen wirken sich bekanntermaßen
sehr stark auf das Immunsystem aus und bewirken
eine entscheidende Abwehrschwäche, sodaß die
Freigabe der Nebennierenwirkstoffe nicht durch
adoptive Immunität sondern vor allem auch die
körpereigene aktivieren wird. Hinzukommen folgende
Überlegungen:

Ein nicht unwesentlicher Grund für die Entnahme
der Spenderlymphe aus dem Ductus thoracicus
erfolgt gemäß der Erfindung deshalb, weil erkannt
wurde, daß dort Hormone der Nebennierenrinde
und des Nebennierenmarks in die Lymphbahnen geleitet
werden, die eine Aktivierung der Lymphozyten
bewirken.[7]

[6] Bier, Götze, u. a.:"Experimentelle und klinische
Immunologie", Springer Verlag 1979 Seite 342 fl.

[7] Selzer: "Die lymphogenen Erkrankungen innerer Organe
und des zentralen Nervensystems". Verlag Laub GmbH & Co,KG
Elztal, 1977, Seite 89 fl.

Hieraus ergab es sich als vorteilhaft, dem erfindungsgemäßen Heilmittel Extrakte von Nebennieren und Nebennierenrinde zuzusetzen. Dies erschien vor allem
bei Krebserkrankungen wichtig, weil bei diesen die
Nebenniere und besonders deren Rinde selbst erkrankt,
sodaß durch deren Funktionsausfall der Patientenlymphe
die Nebennierenwirkstoffe nicht mehr oder nicht
in ausreichendem Maße zugeführt werden.Bei Tierversuchen
konnte durch markierte Lymphozyten nachgewiesen
werden, daß es bei Krebserkrankungen zu solchen
Erkrankungen der Nebenniere kommt. Bei Lymphstauungen,
wie sie bei Krebserkrankungen eintreten, kann es zu
Rückstau der immunologisch-toxischen Lymphe in den
Nebennieren und dadurch zu deren Erkrankung kommen,
die zu Störungen der Hormonproduktion der Nebennieren
führen.

Die Gründe für die nachgewiesene Wirksamkeit des
erfindungsgemäßen Mittels bei Autoimmunkrankheiten
konnte bisher nicht aufgeklärt werden, vor allem
weil der diese Krankheiten verursachende Mechanismus
sich noch weitgehend dem Verständnis entzieht.
Es muß aber angenommen werden, daß die Zuführung
von gesunden Lymphozyten das gestörte Gleichgewicht
zwischen angreifenden Zellen und Suppressorzellen
wieder herzustellen vermag. Es muß vermutet werden,
daß hier wiederum der "allogene Effekt" eingreift[5].

- 17 -

Im folgenden werden einzelne Anwendungsbeispiele
beschrieben:

Der Anwendung der im nachfolgenden beschriebenen
Therapie auf Humanmedizin sind umfangreiche systematische
Versuche mit Tieren, insbesondere Kaninchen vorausgegangen, die zu reproduzierbaren überraschenden Heilerfolgen, insbesondere bei Krebserkrankungen geführt
hatten. Die drei nachstehend beschriebenen Fälle sind
zwar zunächst nur Einzelfälle, bei denen das erfindungsgemäße Heilmittel als letzte Möglichkeit eingesetzt
wurde. Es ergaben sich aber eindeutig den Tierversuchen
entsprechende Ergebnisse:

1. Der Patientin wurde im Alte von 20 Jahren ein
malignes Melanom im Bereich der Brustwirbelsäule
operativ entfernt. Drei Jahre später wurden zwei
Melanommetastasen im Gehirn durch Computer-Tomographie
festgestellt, weitere zwei Jahre später insgesamt
drei solche Metastasen. Da sich im folgenden Jahr bei
der bisherigen Behandlung keine Besserung zeigte,
wurde das erfindungsgemäße Heilmittel eingesetzt.
Es wurden im ganzen paravertebrale intramuskuläre
Injektionen in einem Zeitraum von 38 Tagen gegeben,
die zu einer merklichen Besserung führten. Die Sehkraft war wieder normal, kein Doppelsehen oder Flimmern,
die bestehende Schlundlähmung war behoben, ebenso
Geruchs-, Geschmacks- und Gehörstörungen. Zeitweilige
Ataxien waren nicht mehr aufgetreten, die FWF-
Werte bewegten sich im Normalbereich, woraus geschlossen werden konnte, daß kein Gehirnödem bestand.

ORIGINAL

- 18 -

Die Patientin war so weit hergestellt, daß die Behandlung unterbrochen werden konnte. Bemerkenswert
ist, daß weder Sprach- noch Gedächtnisstörungen
noch Bein- oder Armlähmungen auftraten, wie sie
bei Gehirntumoren zu erwarten sind. Der Tod der
Patientin trat durch einen zerebralen Insult infolge
nekrotischen Zerfalls im Bereich der Hirntumoren
sehr bald nach der letzten Behandlung ein. Der
Obduktionsbefund zeigte, daß, obwohl drei Gehirnmetastasen vorhanden waren mit Ausnahme von zwei
Metastasen in der Leber in den übrigen Organen keine
einzige gefunden werden konnte. Die eine Metastase
im Leberparenchym war erbsengroß, die andere haselnußgroß. Sie ergaben folgenden Befund:

> Leber: Zentral ausgedehnt nekrotisch zerfallene
> Metastasen des seit Jahren bekannten
> malignen Melanoms. Melaninhaltige und
> intakte Tumorzellen sind nur noch in einem
> schmalen Randbezirk erkennbar. Die Metastasen
> sind durch einen teils breiteren, teils
> schmäleren Bindegewebsstreifen gegenüber
> dem umgebenden Leberparenchym abgegrenzt.
> Die Knoten sind in sich geschlossen, ein
> diffuses und grenzenloses infiltrierendes
> Wachstum ist nicht erkennbar. Leichte,
> grob- bis mitteltropfige, vornehmlich
> läppchenzentral etablierte Leberepithel-
> verfettung, wenige bis mäßig reichlich
> vorhandene sog. Kollapsstraßen des Leber-
> parenchyms.

Die Lymphknoten zeigten eine Vergrößerung mit deutlicher
Hyperämie, was auf eine erhöhte Aktivität des Lymphsystems schließen läßt.

0068513

2. Der Patient litt in einem sehr fortgeschrittenen
Stadium an amyotrophischer Lateralsklerose, deren
Ursache unbekannt ist, die als unheilbar gilt und
in zwei bis vier Jahren zum Tod durch Atemlähmung
führt. Die Beweglichkeit der Beine und des Schultergürtels waren bereits durch Muskellähmungen sehr eingeschränkt, daneben bestanden bulbärparalytische
Beschwerden. Die Beine konnten nur bis zu 60° angehoben werden. Die Arme hingen schlaff herunter,
der Patient mußte gefüttert werden. Schreiben war
unmöglich. In einer Zeit von nicht ganz drei Monaten
wurden im ganzen 10 Injektionen des erfindungsgemäßen
Heilmittels in die vertebrale Muskulatur in der
Höhe des Halsmarkes und in die Glutäusmuskulatur in
der Höhe des lumbalen Marks gegeben. Es trat eine
Stärkung der bereits atrophischen Muskulatur und
bei besserer Beweglichkeit eine gute Durchblutung
der Arme und Beine ein. Die Sprachstörungen sind zurückgegangen und die Sprache ist bedeutend deutlicher.
Verschlucken, das vorher sehr häufig war, tritt nur
noch selten auf. Die Schleimbildung in der Lunge
ist gut zurückgegangen und kann leicht beseitigt
werden, was vorher nicht möglich war und es besteht
ein guter Allgemeinzustand und gute Aussichten auf
weitere Besserung.

3. Der Patient leidet an Multipliskerose des II. bis
III. Grades. Infolge einer Kriegsverletzung bestand
eine chronische großflächige (ca. 20 x 10 cm) Entzündung der rechten unteren Bauchmuskulatur mit
drei Darmfisteln, die trotz 15-maliger Operation innerhalb von 38 Jahren nicht beseitigt werden konnten.

BAD ORIGINAL

- 20

Nach einer Behandlung mit 10 intramuskulären Injektionen des erfindungsgemäßen Heilmittels sind folgende Ergebnisse eingetreten:

1. Die Blutsenkung hat sich innerhalb von vier Wochen, in der ersten Woche mit 50/100, in der zweiten Woche mit 70/125, in der dritten Woche mit 75/130*) auf 17/46 in der vierten Woche gebessert.

2. Die Gefühlsstörung, die in der rechten Hand vorhanden war, ist verschwunden.

3. Die Schwäche der Beinmuskulatur hat sich wesentlich gebessert.

4. Keine Gleichgewichtsstörungen oder Unsicherheiten beim Treppensteigen.

5. Er kann jetzt 3 - 4 km auf der Straße gehen.

6. Die Darmfisteln sind innerhalb von vier Wochen vollkommen abgeheilt.

Das nachfolgende Beispiel bezieht sich auf die für das erfindungsgemäße Verfahren angewendete Filtrationstechnik:

4. Die aus dem Ductus thoracicus entnommene Lymphe wird zur Gewinnung eiweißfreier Ultrafiltrate durch Mikrokollodiumhülsen der Firma Sartorius in Göttingen SM 13202 Zellulosennitrat gewonnen. Das Fassungsvermögen der Hülse war 1 ml maximal, der Betriebsdruck war 0,3 bis 0,6 bar. Die Abmessungen der Hülse waren ca. 22 mm Länge und 8,4 mm innerer Durchmesser. Die mittlere Porengröße war 8 µm,

BAD ORIGINAL

- 21 -

das Molekulargewicht betrug 12.400. Zurückgehalten
werden alle Substanzen und Zellen grobmolekularer Art.

Durch dieses Filtern wurden die Lymphflüssigkeit
und die Extrakte der Nebennieren des Spendertieres
filtriert. Erhalten wurde eine klare Flüssigkeit,
in der Eiweißkörper und andere grobmolekulare
Substanzen nicht mehr vorhanden waren.

Weder bei Versuchstieren noch bei Menschen zeigten
sich nach intravenöser, subcutaner und intramuskulärer
Injektion klinische toxologische Symptome.

BAD ORIGINAL

HUBERT FREIHERR VON WELSER

RECHTSANWALT

ZUGELASSEN AN DEN LANDGERICHTEN MÜNCHEN I UND II.
AM OBERLANDESGERICHT MÜNCHEN UND AM
BAYERISCHEN OBERSTEN LANDESGERICHT

DANZIGER STRASSE 15
8000 MÜNCHEN 40
TELEFON (089) 36 70 23

0068513

1. Juli 198

Heilmittel aus Lymphe von Säugetieren

Anmelder: Dr. med Hans Selzer

Schönmünzach

7292 Baiersbronn 9

Patentansprüche:

1. Heilmittel aus Lymphe von Säugetieren für
die Humantherapie sowie Verfahren zu seiner
Herstellung,
dadurch gekennzeichnet, daß es aus aus der Lymphe
des Spendertieres gewonnener von allen einen Filter
von 8 μm mittlerer Porenweite nicht passierenden
grobmolekularem und zellulärem Material gereinigten
Lymphflüssigkeit und nur den im Ductus thoracicus
des Spendertieres rezirkulierenden kleinen
Lymphozyten besteht.

BANKKONTO: DEUTSCHE BANK AG MÜNCHEN 22/26 843 (BLZ 700 700 10) · POSTSCHECKKONTO: MÜNCHEN 1999 94-803
ANDERKONTO: DEUTSCHE BANK AG MÜNCHEN 22/26 850 (BLZ 700 700 10)

2. Heilmittel nach Anspruch 1,
dadurch gekennzeichnet, daß ihm die ausfiltrierten
Extrakte der Nebenniere des gleichen Spendertieres
zugesetzt sind.


3. Verfahren zur Herstellung des Heilmittels nach
Anspruch 1 und 2,
dadurch gekennzeichnet, daß die Lymphe aus dem
Ductus thoracicus des Spendertieres entnommen
und durch einen Filter mit mittlerer Porenweite
von 8 µm bei einem Druck von 0,3 bis 0,6 bar
filtriert wird.

4. Verfahren nach Anspruch 3,
dadurch gekennzeichnet,  daß die Extrakte der
Nebennieren des gleichen Spendertieres durch ein
alle grobmolekularen und zellulären Substanzen
zurückhaltendes Filtern gefiltert und die
so gereinigten Extrakte dem Heilmittel zugesetzt
werden.

5. Heilmittel nach Anspruch 1, 2,
dadurch gekennzeichnet, daß es zur intravenösen,
intramuskulären oder subcutanen Injektion für
die Therapie von Krebserkrankungen oder von
Autoimmunerkrankungen verwendet wird.

Europäisches
Patentamt

**EUROPÄISCHER RECHERCHENBERICHT**

## EINSCHLÄGIGE DOKUMENTE

| Kategorie | Kennzeichnung des Dokuments mit Angabe. soweit erforderlich. der maßgeblichen Teile | Betrifft Anspruch | KLASSIFIKATION DER ANMELDUNG (Int. Cl. 3) |
|---|---|---|---|
| Y | CHEMICAL ABSTRACTS, Band 94, Nr. 7, 16. Februar 1981, Seite 259, Nr. 43697r, Columbus, Ohio, USA & JP - A - 80 135 750 (ASAHI CHEMICAL INDUSTRY CO., LTD.) 22-10-1980 * Zusammenfassung * | 1,3 | A 61 K 35/26 // (A 61 K 35/26 A 61 K 35/23 ) |
| | --- | | |
| Y | CHEMICAL ABSTRACTS, Band 94, Nr. 7, 16. Februar 1981, Seite 260, Nr. 43698s, Columbus, Ohio, USA & JP - A - 80 135 749 (ASAHI CHEMICAL INDUSTRY CO., LTD.) 22-10-1980 * Zusammenfassung * | 1,3 | |
| | --- | | |
| Y | CHEMICAL ABSTRACTS, Band 94, Nr. 7, 16. Februar 1981, Seite 260, Nr. 43700m, Columbus, Ohio, USA & JP - A - 80 136 955 (ASAHI CHEMICAL INDUSTRY CO., LTD.) 25-10-1980 * Zusammenfassung * | 1,3 | |
| | --- | | RECHERCHIERTE SACHGEBIETE (Int. Cl. 3) |
| Y | BIOLOGICAL ABSTRACTS, Band 64, 1977, Seiten 155-162, Nr. 20878, Philadelphia - Pennsylvania, USA J.A. MAJESKI et al.: "Architectural alteration of lymphatic tissue produced by extracorporeal thoracic duct filtration" & J. SURG. ONCOL 9(2), 155-162, 1977 * Zusammenfassung * | 1,3 | A 61 K |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 24-09-1982 | MARIE A.L.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**EUROPÄISCHER RECHERCHENBERICHT**

0068513
Nummer der Anmeldung

EP 82 10 5888

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | BIOLOGICAL ABSTRACTS, Band 56, Nr. 1, 1973, Seiten 255-262, Nr. 2543, Philadelphia - Pennsylvania, USA C.T. FITTS et al.: "The efficacy of closed-circuit extracorporeal filtration of thoracic duct lymph as a means of lymphocyte depletion" & CLIN. EXP. IMMUNOL. 12(2), 255-262, 1972 * Zusammenfassung * | 1,3 | |
| | --- | | |
| Y | IVAN ROITT: "Essential immunology", 4. Auflage, 1980, Seiten 51,52, Blackwell Scientific Publications, Oxford, London * Seiten 51,52 * | 1,3 | |
| | ----- | | RECHERCHIERTE SACHGEBIETE (Int. Cl. ³) |

Der vorliegende Recherchenbericht wurde für alle Patentansprüche erstellt.

| Recherchenort DEN HAAG | Abschlußdatum der Recherche 24-09-1982 | Prüfer MARIE A.L.L. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82